(19) Europäisches Patentamt
European Patent Office
Office européen des brevets

(11) Veröffentlichungsnummer: 0 011 735
B1

(12) # EUROPÄISCHE PATENTSCHRIFT

(45) Veröffentlichungstag der Patentschrift: 24.08.83

(51) Int. Cl.$^3$: A 61 K 6/00

(21) Anmeldenummer: 79104334.2

(22) Anmeldetag: 06.11.79

(54) **Röntgenopake Dentalwerkstoffe auf Basis von organischen Kunststoffen in pastöser Form.**

(30) Priorität: 24.11.78 DE 2850918

(43) Veröffentlichungstag der Anmeldung:
11.06.80 Patentblatt 80/12

(45) Bekanntmachung des Hinweises auf die Patenterteilung:
24.08.83 Patentblatt 83/34

(84) Benannte Vertragsstaaten:
AT BE CH DE FR GB IT LU NL SE

(56) Entgegenhaltungen:
FR - A - 1 052 224
FR - A - 2 028 274
FR - A - 2 370 467
GB - A - 962 221
US - A - 3 265 202
US - A - 3 471 596

(73) Patentinhaber: BAYER AG
Zentralbereich Patente, Marken und Lizenzen
D-5090 Leverkusen 1, Bayerwerk (DE)

(72) Erfinder: Walkowiak, Michael, Dr.
Albertus-Magnus-Strasse 10
D-5090 Leverkusen 1 (DE)
Erfinder: Podszun, Wolfgang, Dr.
Wolfskaul 4
D-5000 Köln 80 (DE)
Erfinder: Leusner, Bernhard, Dr.
Grüner Weg 148
D-5090 Leverkusen (DE)
Erfinder: Süling, Carlhans, Dr.
Carl-Leverkus-Strasse 10
D-5074 Odenthal (DE)
Erfinder: Schulz, Hans Hermann
Am Neulandkreuz 23
D-5653 Leichlingen (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 011 735 B1

# Röntgenopake Dentalwerkstoffe auf Basis von organischen Kunststoffen in pastöser Form

Die Verwendung von röntgenopaken Dentalwerkstoffen für die Herstellung von Zahnfüllungen ist bekannt. Diese als Composites bezeichneten Materialien enthalten als röntgenopaken Füllstoff feinteilige Glaspulver. Nachteilig bei diesen Materialien ist die fehlende Polierbarkeit mit allen daraus resultierenden bekannten Nachteilen. Außerdem haben sie wegen ihrer Konsistenz und Klebrigkeit weitere anwendungstechnische und klinische Nachteile.

Das Einbringen des Materials in die Kavität erfolgt durch Einstreichen, häufig wird die eingebrachte Masse, bedingt durch das Haften am Füllinstrument, teilweise nach dem Einfüllen in die Kavität von der Kavitätenwandung abgezogen. Diese Erscheinung ist in der Regel vom Zahnarzt nicht feststellbar und führt daher zu nicht wandständigen, inkompletten Füllungen mit den bekannten Nachteilen.

Besonders nachteilig wirkt sich das verstärkte Kleben der bisher bekannten Füllmaterialien bei mehrflächigen Kavitäten aus. So ist, wie aus der Amalgamfülltechnik her bekannt, ein einwandfreien Ausfüllen der Kavität nur möglich, wenn ein Füllmaterial portionsweise eingebracht wird. Bei dieser Fülltechnik werden zunächst kleine Portionen wandständig in die Kavitätenwinkel gestopft und anschließend erst die Kavität ausgefüllt. Eine entsprechende Arbeitsweise ist mit den bisher bekannten Kunststoffmaterialien nicht möglich.

Während bei einflächigen Füllungen im Frontzahnbereich die Oberflächengestalt durch Anlegen von Matrizenbändern erzielt wird, bereitet die Formung von okklusalen Flächen mit Materialien, die eine klebende Konsistenz aufweisen, Schwierigkeiten. So war bei den bisher bekannten Materialien eine Formung der Kauflächenbezirke nur in groben Zügen möglich. Nach dem Aushärten war daher üblicherweise eine Formgebung durch rotierende Schleif- und Polierkörper erforderlich. Bekanntlich sind hierbei Verletzungen der benachbarten Schmelzbezirke in der Regel nicht zu vermeiden. Die Folgen hiervon sind Verfälschungen des Kauflächenreliefs und gegebenenfalls okklusale Störungen.

Man hat versucht, die wünschenswerte Oberflächengestalt durch Einstellung einer "schnitzbaren" Eigenschaft zu erzielen. Dieses "schnitzbare" Verhalten tritt jedoch erst dann ein, wenn schon ein gewisser Polymerisationsgrad erreicht ist. Erfolgt in diesem Zustand die Bearbeitung des Füllmaterials, so kann ein Auf- bzw. Einreißen der Füllungsoberfläche und damit eine Schädigung der Füllung nicht ausgeschlossen werden. Diese Risse, erzielt durch "Schnitzen", können Eintrittspforten für Microorganismen und für Farbstoffe mit den bekannten Auswirkungen sein. Darüber hinaus kann das Bearbeiten von schon anpolymerisierten Material, zu Polymerisationsstörungen führen. Gegenstand der Erfindung sind nun röntgenopake Zahnfüllmassen auf Basis von organischen Kunststoffen in pastöser Form, welche dadurch gekennzeichnet sind, daß sie aus

a) einem oder mehreren polymerisierbaren Bindern,
b) einem oder mehreren vernetzten Perlpolymerisaten mit einer mittleren Teilchengröße von 10—100 $\mu$m,
c) einem oder mehreren Röntgenkontrastmitteln, sowie gegebenenfalls
d) nicht röntgenopaken anorganischen Füllstoffen mit einer mittleren Teilchengröße von 1 $\mu$m bis 5 $\mu$m

besteht, wobei der Anteil der Komponente c) 5—45 Gew.-% beträgt und wobei Komponente b) einen anorganischen Füllstoff enthalt.

Dentalmassen, welche vernetzte, Füllstoff-haltige Perlpolymerisate enthalten, sind bisher nicht bekannt geworden. Es wurde überraschenderweise gefunden, daß solche Pasten sich hervorragend als Zahnfüllmaterial eignen.

In US—A—3 265 202 werden Dentalmassen beschrieben, welche ein unvernetztes Perlpolymerisat auf Acrylatbasis enthalten. Anorganische Füllstoffe, die der Masse Röntgenopazität verleihen würden, werden nicht erwähnt. US—A—3 471 596 beschreibt pastöse Dentalwerkstoffe, die polymeres Allylmethacrylat sowie Polymethylmethacrylatperlen als Füllstoff, Pigmente und Polyglycoldimethacrylat enthalten. Auch dieser Druckschrift sind keine vernetzten Perlpolymerisate zu entnehmen; es wird in ihr auch keine Anregung gegeben, Perlpolymerisate einzusetzen, welche einen anorganischen Füllstoff enthalten. Ein Vorteil dieser erfindungsgemäßen Maßnahme liegt darin, daß der anorganische Füllstoff im ausgehärteten Dentalmaterial homogen verteilt ist, was zu verbesserten mechanischen Eigenschaften führt.

Die erfindungsgemäßen Materialien lassen sich darüber hinaus in einer Konsistenz herstellen, die eine Verarbeitung ermöglicht, wie sie in der Amalgamfülltechnik gebräuchlich ist, d.h. sie lassen sich stopfen und modellieren.

Mit den erfindungsgemäßen Materialien gelingt es, aufgrund einer nicht klebenden, festen, stopfbaren Konsistenz, ein- und mehrflächige Kavitäten in mehreren Portionen wandständig aufzufüllen. Durch die besondere Eigenschaft des Materials kommt es beim portionsweisen Stopfen nicht zu Schichtenbildung, das heißt, die einzelnen Portionen verbinden sich homogen miteinander. Nach dem jeweiligen Einbringen in die Kavität und dem Stopfen bzw. Adaptieren bleibt diese, ohne ihre Form zu verändern am Ort liegen, d.h. sie läßt sich auch nicht elastisch deformieren.

Die Kavitätenfüllung läßt sich ferner, aufgrund der besonderen Konsistenz, mit sogenannten Amalgampistolen durchführen, ohne daß sich das Füllmaterial wieder von der Kavitätenwandung abzieht oder an der Pistolenöffnung haften bleibt.

Die erfindungsgemäßen Materialien zeigen eine durch Instrumente formbare Konsistenz schon unmittelbar nach dem Abmischvorgang. Diese Konsistenz ermöglicht es, daß nach dem Auffüllen der Kavität, mittels geeigneter Instrumente, z.B. aus Kunststoff oder aus Metall, wie sie in der Amalgamfülltechnik verwendet werden, die okkusale individuelle Kauflächengestalt geformt wird.

Die pastösen erfindungsgemäßen Dentalwerkstoffe auf Basis von organischen Kunststoffen gehen durch Aushärtung in feste Körper über. Diese haben den großen Vorteil, radioopak und gut polierbar zu sein.

Zur Herstellung der erfindungsgemäßen Dentalwerkstoffe werden 18 bis 60, vorzugsweise 25—50 Gew.-Teile polymerisierbare Binder, 20—75 vorzugsweise 30—65 Gew.-Teile füllstoffhaltige vernetzte Perlpolymerisate, 5—45 Gew.-Teile Röntgenkontrastmittel, gegebenenfalls bis zu 28 Gew.-% feinteiliger, nicht röntgenopaker organischer Füllstoff und 0,01 bis 5 Gew.-Teile Starterzusätze zu einer Paste vermischt.

Zur Erleichterung der Pastenfertigung können Inhibitoren oder Lichtschutzmittel eingesetzt werden Für bestimmte Indikationen kann es angezeigt sein, zusätzlich Farbstoffe zuzusetzen.

Als polymerisierbare Binder eignen sich die Ester der Methacrylsäure von einwertigen und mehrwertigen Akloholen gegebenenfalls im Gemisch mit anderen Vinylmonomeren. Besonders günstig ist es, wenn der Gehalt an Methacrylsäureestern über 80% beträgt.

Als geeignete Ester der Methacrylsäure seien beispielsweise aliphatische und cycloaliphatische Ester genannt, wie Methylmethacrylat, Ethylmethacrylat und Cyclohexylmethacrylat.

Besonders gut geeignet sind ferner Ester von mehrwertigen Alkoholen mit einem Molekulargewicht von 190—10000, insbesondere Ester von 2 und 3-wertigen Alkoholen mit einem Molekulargewicht im Bereich von 190 bis 800, wie z.B. Ethylenglykoldimethacrylat, Triethylenglykoldimethylmethacrylat, Neopentylglykoldimethacrylat oder Trimethylolpropantrimethacrylat, desweiteren Urethan und Ureidopolymethacrylate, die durch Umsetzung von Hydroxyalkylmethacrylat bzw. Aminoalkylmethacrylaten mit Polyisocyanaten zugänglich sind, z.B. die Verbindung

$$CH_2{=}\overset{\overset{CH_3}{|}}{C}-COO-(CH_2)_2-OOC-NH-(CH_2)_6-NH-COO-(CH_2)_2-OOC-\overset{\overset{CH_3}{|}}{C}{=}CH_2$$

Sehr gute Pasten erhält man, wenn als Binder zumindest in Anteilen Verbindungen vom Typ des Bis-GMA der Formel

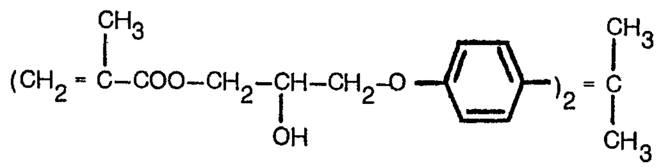

eingesetzt werden.

Zahnfüllmassen mit guter Konsistenz und einem hohen Niveau der mechanischen Festigkeit werden besonders dann erhalten, wenn man als Binder Mischungen auf verschiedenen Methacrylsäureestern verwendet, z.B. Mischungen von 20—70 Gew.-Teilen Bis GMA und 80—30 Gew.-Teilen Triethylenglykoldimethacrylat.

Die zur Pastenherstellung eingesetzten vernetzten Perlpolymerisate sollen zur mehr als 50 Gew.-% aus polymerisierten Methacrylsäureestern vorzugsweise Methacrylsäuremethylesterbestehen. Als vernetztend wirkende Monomere eignen sich die mit Methylmethacrylat copolymerisierbaren Polyvinylverbindungen, wie beispielsweise Äthylenglykoldimethacrylat, Divinylbenzol, wobei der Vernetzeranteil 2 bis 35 Gew.-% des Monomergemisches betragen sollte. Neben dem Vernetzer können weitere Monomere in das Perlpolymerisat einpolymerisiert sein, um beispielsweise das Quellverhalten des Perlpolymerisats zu beeinflussen oder um die mechanischen Eigenschaften des ausgehärteten Dentalkunststoffs zu modifizieren. Die mittlere Korngröße der eingesetzten Perlpolymerisate soll zwischen 10 und 100 $\mu$m liegen besonders günstig ist der Bereich von 10 bis 70$\mu$m.

Erfindungsgemäß werden Perlpolymerisate eingesetzt, die einen anorganischen Füllstoff enthalten. Für die Pastenzubereitung besonders gut geeignet sind Füllstoff-haltige Polymerisatperlen auf Basis von polymerisierten (Meth)-acrylsäureestern mit einer Viskosität von 0,1 bis 10 Pa.s. Die Herstellung von mit anorganischen Füllstoffen gefüllten Perlpolymerisaten wird in der europäischen Patentanmeldung 79104253.4 beschrieben.

Die erfindungsgemäße Verwendung von Füllstoff-haltigen Perlpolymerisaten ist besonders vorteilhaft, da auf diese Weise Dentalwerkstoffe erhalten werden können, bei denen sowohl das Perlpolymerisat als auch die Perlzwischenräume gleichermaßen anorganischen Füllstoff enthalten, wodurch ein hohes Maß an Homogenität im ausgehärtetem Werkstoff erreicht wird.

Als Röntgenkontrastmittel können die in der Medizin bekannten Röntgenkontrastmittel eingesetzt werden, gut geeignet sind feste schwerlösliche Schwermetallverbindungen. Sie müssen in besonders feiner Verteilung vorliegen, vorzugsweise mit einer mittleren Teilchengröße von 1 nm bis 5 μm.

Besonders gut eignen sich Bariumverbindungen, z.B. Bariumsulfat, Bariumfluorid und Bariumsilikat. Aber auch Verbindungen von Wismut, z.B. Wismutoxynitrat, Zirkon, z.B. Zirkondioxid und Lanthan, z.B. Lanthanoxid, sowie Verbindungen von Thorium und den seltenen Erdmetallen sind geeignet. Darüber hinaus können anorganische und organische Jodverbindungen als Röntgenkontrastmittel verwendet werden.

Die eingesetzte Menge Röntgenkontrastmittel kann in weiten Grenzen je nach den klinischen Bedürfnissen variiert werden. Ein guter Röntgenkontrast wird z.B. bei einem Gehalt von 15—40, vorzugsweise 20—30 Gew.-% Bariumsulfat erreicht.

Als feinteiliger, nicht röntgenopaker anorganischer Füllstoff für die erfindungsgemäßen Dentalwerkstoff sind in erster Linie Siliciumdioxid, Aluminiumdioxid, Silikate und Silikatgläser geeignet, sofern ihre mittlere Teilchengröße im Bereich vom 1 mμ bis 1 μ liegt. Besonders günstig ist die Verwendung von flammpyrolytisch gewonnenem amorphen Siliciumdioxid, und zwar vorzugsweise von amorphem Siliciumdioxid mit einer Primärteilchengröße von 5—30 nm und einer spezifischen Oberfläche gemessen nach BET von 40—400 $m^2/g$.

Der feinteilige anorganische Füllstoff kann mit Haftvermittleren beispielsweise mit Vinylmethoxysilan oder Trimethoxy-(3-methacryloyloxipropyl)-silan nachbehandelt werden, um den Verbund zwischen anorganischen Füllstoff und organischer Matrix zu verbessern, jedoch ist dieser Nachbehandlungsschritt zur Herstellung der erfindungsgemäßen Dentalwerkstoffe nicht unbedingt erforderlich.

Als Starterzusätze können die üblichen Startersysteme verwendet werden, d.h. Radikale, Anionen oder Kationen liefernde Systeme, die eine radikalische, anionische oder kationische Polymerisation auslösen können. Im Falle von Radikale liefernden Systemem sind Peroxide oder aliphatische Azoverbindungen besonders geeignet, beispielsweise Benzoylperoxid, Lauroylperoxid oder Azoisobuttersäuredinitril, die üblicherweise in Mengen von 0,1 bis 5 Gew.-% eingesetzt werden. Währen die Härtung bei erhöhter Temperatur allein durch Peroxide oder andere Radikalstarter durchgeführt wird, ist zur Härtung bei Raumtemperatur ein Zusatz von Beschleunigern, vorzugsweise aromatischen Aminen notwendig. Geeignete Beschleuniger sind N-N-substituierte Toluidine und Xylidine, wie NN-Dimethyl-p-Toluidin oder NN-Bis-(2-hydroxyethyl)-xylidin. Gute Aushärtung erzielt man mit 0,5—3% Aminzusatz. Eine günstige Darbietungsform für einen Peroxid/Beschleuniger aktiviertes System ist die 2-Pasten-Form, wobei eine Paste den Radikalstarter und die andere den Beschleuniger enthält und die Aushärtung durch Mischen beider Pasten eingeleitet wird.

Auch eine Aushärtung unter Verwendung von UV-Licht oder sichtbarem Licht bei geeigneter Sensibilisierung ist eine sehr gute Methode. Geeignete Photoinitiatoren sind beispielsweise Benzophenon und seine Derivate, Benzoin und seine Derivate, wie Benzoinether, Anthrochinone und aromatische Disulfide.

### Beispiel 1

*Herstellung eines mit Bariumsulfat gefüllten Perlpolymerisats*

*Polymerisation*

| | | |
|---|---|---|
| Reaktionsgefäß | : | 6-Liter-Autoklav mit Doppelankerrührer |
| Lösung I | : | 2500 ml dest. Wasser |
| (Dispergator-Lösung) | | 500 ml, 7,5%ige wäßrige Lösung des Copolymerisats aus 1 Gew.-Teil Methacrylsäure und 1 Gew.-Teil Methylenmethacrylat mit pH = 6 und der Viskosität: 3650 mPas. |

Lösung II:

220 g Methylmethacrylat
30 g Ethylenglykoldimethacrylat
50 g Polymethylmethacrylat
([η] = 1,05 in Chloroform)
100 g Bariumsulfat (Riedel de Haën AG)

Die Komponenten der Lösung II werden unter Ausschluß von Luftsauerstoff in das Reaktionsgefäß gegeben und solange bei Raumtemperatur verrührt, bis das Polymerisat gelöst ist. Die erhaltene Mischung wird mit 1,1 g Benzoylperoxid versetzt und weitere 15 Minuten gerührt. Danach wird Lösung I (Dispergatorlösung) auf einmal zugegeben und die Rührgeschweindigkeit auf 400 UpM erhöht.

Man erhitzt die gebildete Suspension auf 80°C und kühlt bei einsetzender exothermer Reaktion so stark ab, daß die Temperatur unter 85°C gehalten wird. Nach dem Abklingen der Reaktion wird der Ansatz unter weiterem Rühren 2 Stunden lang auf 85°C gehalten.

*Aufarbeitung*

Der Ansatz wird abgeblasen und mit dest. Wasser auf 10 l verdünnt. Nach der Zugabe von 180 g Eisessig wird 15 Minuten auf 90—100°C aufgeheizt. Das ausfallende Perlpolymerisat wird nach Erkalten abgesaugt, durch dreimaliges Aufrühren in je 5 l dest. Wasser gewaschen und bei 60°C getrocknet.

Ausbeute 275 g, Ba $SO_4$-Gehalt 28,8%, mittlerer Perldurchmesser ca. 45 $\mu$m.

Beispiel 2

*Erfindungsgemäßer, pastöser Werkstoff*

*A) Peroxidpaste*

105 g Perlpolymerisat gemäß Beispiel 1,
10 g silanisiertes amorphes Siliciumdioxid (BET-Oberfläche 170 $m^2/g$)
26 g Bariumsulfat,
42 g Bis-GMA (Nupol 46—4005 der Firma Freeman Chemical) und
22 g Triäthylenglykoldimethacrylat sowie
1,1 g Benzoylperoxid

Die einzelnen Komponenten werden in einen Kneter gegeben und 60 Minuten lang intensive geknetet, wobei während der letzten 10 Minuten ein Vakuum von ca. 20 Torr angelegt wird. Man erhält auf diese Weise eine knetbare Masse von besonders fester Konsistenz.

*B) Aminpaste*

Perlpolymerisat, amorphes Siliciumdioxid, Bariumsulfat und Urethandimethacrylat werden in den gleichen Mengen wie bei der Peroxidpaste eingesetzt und verarbeitet. Anstelle des Peroxids werden 0,9 g N,N-Bis-(2-hydroxypropyl)-3,5-dimethylanilin eingesetzt.

*C) Pastöse Masse zum Füllen von Zähnen*

Gleiche Teile (z.B. je 200 mg) der Amin- und Peroxidpaste werden 30 Sekunden lang intensive gemischt. Die erhaltene Mischung ist in Hervorragender Weise als Zahnfüllmaterial geeignet. Sie härtet in wenigen Minuten unter geringem Polymerisationsschrumpf aus.

**Patentansprüche**

1. Röntgenopake Zahnfüllmasse auf Basis von organischen Kunststoffen in pastöser Form, dadurch gekennzeichnet, daß sie aus

a) einem oder mehreren polymerisierbaren Bindern,
b) einem oder mehreren vernetzten Perlpolymerisaten mit einer mittleren Teilchengröße von 10—100 $\mu$m,
c) einem oder mehreren Röntgenkontrastmitteln, sowie gegebenenfalls
d) nicht röntgenopaken anorganischen Füllstoffen mit einer mittleren Teilchengröße von 1 nm bis 5 $\mu$m

besteht, wobei der Anteil der Komponente c) 5—45 Gew.-% beträgt, und wobei Komponente b) einen anorganischen Füllstoff enthält.

2. Röntgenopake Zahnfüllmasse nach Anspruch 1, dadurch gekennzeichnet, daß Komponente a) zu mehr als 80 Gew.-% aus Estern der Methacrylsäure besteht.

3. Röntgenopake Zahnfüllmasse nach Anspruch 1 oder 2, dadurch gekennzeichnet, daß Komponente a) zu mindestens 50 Gew.-% aus Estern der Methacrylsäure mit 2 oder mehr polymerisierbaren Doppelbindungen besteht.

4. Röntgenopake Zahnfüllmasse nach Anspruch 1 bis 3, dadurch gekennzeichnet, daß Komponente b) zu mehr als 50 Gew.-% aus Estern der Methacrylsäre aufgebaut ist.

5. Röntgenopake Zahnfüllmasse nach Anspruch 1 bis 4, dadurch gekennzeichnet, daß Komponente c) auf festen, schwerlöslichen, anorganischen Substanzen mit einer mittleren Teilchengröße von 1 nm bis 5 $\mu$m besteht.

6. Röntgenopake Zahnfüllmasse nach Anspruch 1 bis 5, dadurch gekennzeichnet, daß Komponente d) in einer Menge bis zu 28 Gew.- vorliegt.

7. Röntgenopake Zahnfüllmasse nach Anspruch 1 bis 6, dadurch gekennzeichnet, daß Komponente d) aus amorpher Kieselsäure besteht.

8. Röntgenopake Zahnfüllmasse nach Anspruch 1 bis 7, dadurch gekennzeichnet, daß der Anteil der Komponenten b), c) und d) zusammen 51 bis 82 Gew.-% beträgt.

9. Röntgenopake Zahnfüllmasse nach Anspruch 1 bis 8, dadurch gekennzeichnet, daß Komponente b) als anorganischen Füllstoff Bariumsulfat enthält.

**Revendications**

1. Masse d'obturation dentaire opaque aux rayons X à base de résines synthétiques organiques à l'état pâteux, caractérisée en ce qu'elle consiste en
   a) un ou plusieurs liants polymérisables,
   b) un ou plusieurs polymères en perles réticulés à une dimension de particule moyenne de 10 à 100 microns,
   c) un ou plusieurs agent de contraste aux rayons X, et le cas échéant,
   d) des matières de charge minérales non opaques aux rayons X à une dimension de particule moyenne de 1 nm à 5 $\mu$m, la proportion du composant c) étant de 5 à 45% en poids et le composant b) contentant une matière de charge minérale.
2. Masse d'obturation dentaire opaque aux rayons X selon la revendication 1, caractérisé en ce que le composant a) consiste pour plus de 80% en poids en esters de l'acide méthacrylique.
3. Masse d'obturation dentaire opaque aux rayons X selon la revendication 1 ou 2, caractérisée en ce que le composant a) consiste pour au moins 50% en poids en esters de l'acide méthacrylique contenant deux doubles liaisons polymérisables ou plus.
4. Masse d'obturation dentaire opaque aux rayons X selon les revendications 1 à 3, caractérisée en ce que le composant b) est constitué pour plus de 50% en poids d'esters de l'acide méthacrylique.
5. Masse d'obturation dentaire opaque aux rayons X selon les revendications 1 à 4, caractérisée en ce que le composant c) consiste en substances minérales solides peu solubles à une diamètre de particule moyen de 1 nm à 5 microns.
6. Masse d'obturation opaque aux rayons X selon les revendications 1 à 5, caractérisée en ce que le composant d) est présent en quantité allant jusqu'à 28% en poids.
7. Masse d'obturation dentaire opaque aux rayons X selon les revendications 1 à 6, caractérisée en ce que le composant d) consiste en silice amorphe.
8. Masse d'obturation dentaire opaque aux rayons X selon les revendication 1 à 7, caractérisée en ce que la proportion des composants b), c) et d), ensemble, représente de 51 à 82% en poids.
9. Masse d'obturation dentaire opaque aux rayons X selon les revendications 1 à 8, caractérisée en ce que le composant b) contient du sulfate de baryum en tant que matière de charge minérale.

**Claims**

1. Dental filling composition which is opaque to X-rays and is based on organic plastics in paste form, characterised in that it consists of
   a) one or more polymerisable binders,
   b) one or more crosslinked bead polymers having an average particle size of 10—100 $\mu$m,
   c) one or more X-ray contrast media and, if approppriate,
   d) inorganic fillers which are not opaque to X-rays and have an average particle size of 1 nm to 5 $\mu$m,

the proportion of component c) beign 5—45% by weight, and component b) containing an inorganic filler.

2. Dental filling composition which is opaque to X-rays, according to Claim 1, characterised in that component a) consists to the extent of more than 80% by weight of esters of methacrylic acid.
3. Dental filling composition which is opaque to X-rays, according to Claim 1 or 2, characterised in that component a) consists to the extent of at least 50% by weight of esters of methacrylic acid with 2 or more polymerisable double bonds.
4. Dental filling compositions which is opaque to X-rays, according to Claim 1 to 3, characterised in that component b) is made up to the extent of more than 50% by weight of esters of methacrylic acid.
5. Dental filling composition which is opaque to X-rays, according to Claim 1 to 4, characterised in that component c) consists of solid, sparingly soluble inorganic substances with an average particle size of 1 nm to 5 $\mu$m.
6. Dental filling composition which is opaque to X-rays, according to Claim 1 to 5, characterised in that component d) is present in an amount of up to 28% by weight.
7. Dental filling composition which is opaque to X-rays, according to Claim 1 to 6, characterised in that component d) consists of amorphous silicic acid.
8. Dental filling composition which is opaque to X-rays, according to Claim 1 to 7, characterised in that the proportion of components b), c) and d) together is 51 to 82% by weight.
9. Dental filling composition which is opaque to X-rays, according to Claim 1 to 8, characterised in that component b) contains barium sulphate as the inorganic filler.